# EUROPÄISCHE PATENTSCHRIFT

(11) **EP 2 055 270 B1**
(45) Veröffentlichungstag und Bekanntmachung des Hinweises auf die Patenterteilung: **11.07.2012**
(21) Anmeldenummer: 08166891.5
(22) Anmeldetag: 17.10.2008
(51) Int. Cl.: A61F 2/30, A61F 2/00, A61F 2/46

(54) **Handhabungswerkzeug für ein medizinisches Implantat, medizinisches Implantat und Handhabungswerkzeug und Implantat umfassender Teilesatz**
Handling tool for a medical implant, medical implant and kit including a handling tool and an implant
Outil de manipulation manuel pour un implant médical, implant médical et ensemble comprenant un outil de manipulation ainsi qu'un implant

(30) Priorität: 30.10.2007 DE 102007052173
(43) Veröffentlichungstag der Anmeldung: 06.05.2009
(73) Patentinhaber: Kraus, Kilian, 97440 Werneck (DE)
(72) Erfinder: Kraus, Kilian, 97440 Werneck (DE)
(74) Vertreter: Mörtel, Alfred

(56) Entgegenhaltungen:
- EP-A- 1 219 266
- EP-A- 1 818 025
- EP-A1- 1 219 266
- EP-A1- 1 818 025
- WO-A-2005/055887
- WO-A-2006/055168
- WO-A-2006/055168
- DE-A1- 19 604 494
- FR-A- 2 663 840

## Beschreibung

Die Erfindung betrifft ein Handhabungswerkzeug für ein medizinisches Implantat, an dessen hinterem Ende ein Griffteil und an dessen vorderem Ende ein Fixierelement zur lösbaren axialen Fixierung des Implantates vorhanden ist. Ein solches Handhabungswerkzeug geht beispielsweise aus der WO 2005/055887 hervor.

Die Erfindung betrifft des weiteren ein medizinisches Implantat. Solche Implantate werden verbreitet als therapeutische Maßnahme zum Ersatz eines beschädigten Körperteils eingesetzt. Neben Implantaten, beispielsweise für Zähne und Gelenke, existieren auch solche zum Ersatz eines beschädigten Wirbelkörpers. Entscheidend für den Erfolg einer Operation ist, dass das Implantat an seinem gewünschten Ort exakt und zuverlässig positionierbar ist.

Schließlich betrifft die Erfindung noch einen Teilesatz, der ein medizinisches Implantat und ein Handhabungswerkzeug umfasst.

Die DE 691 09 768 T2 offenbart ein Handhabungswerkzeug für eine Hüftgelenksprothese. Dieses wird mit Hilfe von Nocken nach der Art eines Bajonettverschlusses in an der Prothese vorhandene Hinterschnitten eingehakt. Zur Drehfixierung des Handhabungswerkzeuges an dem Implantat wird ein Stempel in das Implantat gepresst, so dass die Sperrnocken reibschlüssig in die implantatseitigen Hinterschnitte greifen.

Die DE 196 04 494 A1 offenbart ein weiteres Werkzeug zur Implantierung einer Endoprothese in den Hohlraum eines menschlichen Knochens. Die Prothese wird mit Hilfe eines Stiftes in Axialrichtung an dem Handhabungswerkzeug fixiert. Dieser sich in Axialrichtung erstreckende Stift weist einen Queransatz auf, der bei einer Drehung des Stiftes in einen passenden Hinterschnitt des Implantates greift.

Die EP 1 219 266 A1 bildet die Basis für die Oberbegriffe der Ansprüche 1 und 20 und offenbart ein Operationsinstrument als weiteres Werkzeug zur Positionierung eines Wirbelsäulenimplantates, dieses Werkzeug weist an seinem dem Implantat zugewandten vorderen Ende eine Gabel mit vier Gabelfingern auf. Ein erstes Gabelpaar wird in passgenaue Quernuten des Implantates eingeführt, so dass Implantat und Handhabungswerkzeug reibschlüssig aneinander gehalten sind. Das zweite Gabelpaar verhindert die Verstellung des Implantates während des Einsetzvorganges.

Die WO 2005/055887 offenbart ein Handhabungswerkzeug für ein höhenverstellbares Wirbelsäulenimplantat. Das Handhabungswerkzeug ist zur Verbindung mit dem Implantat so ausgestaltet, dass es in eine entsprechende Öffnung des Implantates passgenau einsteckbar oder einschraubbar ist. Wird das Handhabungswerkzeug in das Implantat eingesteckt, so existiert zwischen den Kontaktflächen der beiden Bauteile ggf. eine reibschlüssige mechanische Verbindung. Eine reibschlüssige Verbindung ist allgemein nicht für hohe Belastungskräfte ausgelegt. Eine Schraubverbindung ist in Axialrichtung des Handhabungswerkzeug, wenn dieses an seinem vorderen Ende ein radial umlaufendes Gewinde trägt, weit höher belastbar. Es ist jedoch wesentlich aufwendiger das verschraubte Implantat von dem Handhabungswerkzeug zu trennen. Die Möglichkeit zur einfachen Trennung von Implantat und Handhabungswerkzeug ist insbesondere deshalb relevant, da die Trennung zwischen Implantat und Handhabungswerkzeug eine mögliche Fehlerquelle bei Operationen darstellt und unnötigen Zeitverlust verursacht.

Aufgabe der vorliegenden Erfindung ist es, ein Handhabungswerkzeug, ein medizinisches Implantat sowie einen Teilesatz anzugeben, wobei eine zuverlässige, kraftschlüssige Verbindung zwischen dem Implantat und dem Handhabungswerkzeug ermöglicht sein soll, die außerdem einfach lösbar ist.

Die vorgenannte Aufgabe wird durch ein Handhabungswerkzeug nach Anspruch 1, ein Implantat nach Anspruch 20 und einen Teilesatz nach Anspruch 26 gelöst.

Erfindungsgemäß weist das Handhabungswerkzeug für ein medizinisches Implantat einen Betätigungsstab auf, an dessen hinterem Ende ein Griffteil und an dessen vorderem Ende ein Fixierelement zur lösbaren axialen Fixierung des Implantates vorhanden ist. Das Fixierelement weist zumindest zwei Sperrbacken auf, die einen Radialabstand zur Mittellängsachse des Betätigungsstabes einhalten. Bezüglich eines sich koaxial zur Mittellängsachse des Betätigungsstabes erstreckenden, gedachten Zylindermantels weisen die Sperrbacken einen gegenseitigen Abstand in Umfangsrichtung auf. Die Sperrbacken des Handhabungswerkzeuges dienen dem Hintergriff wenigstens eines implantatseitigen Gegenelementes. Eine Einrichtung zur Dreharretierung des Handhabungswerkzeuges an einem Implantat umfasst wenigstens ein Arretierelement, welches drehfest mit dem Betätigungsstab verbunden ist. Das Arretierelement ist zwischen einer vorderen Position, seiner Arbeitsstellung, und einer hinteren Position, seiner Ruhestellung, axial verschiebbar an dem Betätigungsstab gehalten. Das Arretierelement wirkt mit einer Anschlagfläche des Implantats drehfest zusammen.

Den erfindungsgemäßen Maßnahmen liegt dabei die folgende Überlegung zu Grunde: Durch Hintergreifen wenigstens eines Implantatseitigen Gegenelementes kann eine einfache axiale Fixierung des Implantates erreicht werden. Da die zumindest zwei Sperrbacken in Umfangsrichtung beabstandet sind, ist eine Verriegelung zwischen dem Handhabungswerkzeug und dem Implantat durch weniger als eine Volldrehung möglich. Das Implantat und das Handhabungswerkzeug können somit schnell und problemlos miteinander verbunden, wie auch voneinander getrennt werden. Das Implantat und das Handhabungswerkzeug können beispielsweise durch eine reibschlüssige Verbindung zwischen den Sperrbacken und einem implantatseitigen Gegenelement drehfest miteinander verbunden werden. Die Möglichkeit zur Dreharretierung des Implantates gegenüber dem Handhabungswerkzeug erweitert dessen Einsatzbereich. Ein solches Handhabungswerkzeug erlaubt eine zuverlässige, exakte Positionierung des Implantates, wobei nunmehr ein Positioniervorgang des Implantates möglich ist, bei dem dieses zusätzlich um die Mittellängsachse des Handhabungswerkzeuges gedreht wird. Das Arretierelement ist derart ausgebildet, dass dieses zwischen einer vorderen Position, seiner Arbeitsstellung, und einer hinteren Position, seiner Ruhestellung, axial verschiebbar und an dem Betätigungsstab gehalten ist. Eine solche Ausgestaltung des Arretierelementes eröffnet die Möglichkeit bei der Herstellung der Axialfixierung das Arretierelement zur Drehfixierung aus dem Bereich des Fixierelementes zu entfernen, so dass die Axialfixierung ungehindert hergestellt werden kann.

Vorteilhafte Ausgestaltungen des erfindungsgemäßen Handhabungswerkzeugs gehen aus den von Anspruch 1 abhängigen Ansprüchen hervor. Dabei kann das Handhabungswerkzeug nach der Erfindung mit den Merkmalen eines, insbesondere mit denen mehrerer Unteransprüche kombiniert werden.

Gemäß einer ersten Ausführungsform ist das Arretierelement in Richtung seiner Arbeitsstellung federbelastet. Die Bedienung des Handhabungswerkzeuges ist nunmehr dadurch verbessert, dass das Arretierelement federbelastet ist, und in seiner Arbeitsstellung einrasten kann. Außerdem kann eine Verrastung der Dreharretierung bei einem derart in Richtung seiner Arbeitsstellung vorgespannten Arretierelement nicht versäumt werden.

Nach einer Ausführungsform weist die Einrichtung zur Dreharretierung ein den Betätigungsstab umschließendes, und axial an diesem verschiebbares Hüllrohr auf. Das vordere Ende des Hüllrohres trägt wenigstens ein fest damit verbundenes Arretierelement. Eine Dreharretierung in Form eines Hüllrohres ist besonders stabil und schützt außerdem das umliegende Gewebe vor Verletzungen durch den Betätigungsstab.

Gemäß einer Weiterbildung ist das Hüllrohr dadurch vorgespannt, dass sich ein Federelement mit seinem hinteren Ende an dem Betätigungsstab und mit seinem vorderen Ende an dem Hüllrohr abstützt. Das Hüllrohr ist also durch das Federelement gegenüber dem Betätigungsstab vorgespannt. Dies gilt natürlich ebenso für das mit dem Hüllrohr verbundene Arretierelement. Die Bedienung des Handhabungswerkzeuges wird dadurch verbessert, dass das an dem Hüllrohr vorhandene Arretierelement durch die Federkraft des Federelementes in sein entsprechendes implantatseitiges Widerlager gedrückt wird.

Gemäß einer weiteren Ausführungsform ist das hintere Ende des Hüllrohres unter Ausbildung eines Gehäuses radial erweitert. In diesem Gehäuse ist das wenigstens eine Federelement angeordnet. Das Hüllrohr kann besonders einfach an dem Gehäuse zurück gezogen werden, was die Bedienung des Handhabungswerkzeuges vereinfacht.

Das Hüllrohr ist, gemäß einer Weiterbildung, verliersicher an dem Betätigungsstab gehalten. Sollte das Hüllrohr während einer Operation versehentlich zu Boden fallen, so ist es aus Gründen der Hygiene notwendig dieses erneut zu desinfizieren. Der Desinfektionsvorgang führt zu einer Unterbrechung der Operation. Es ist daher besonders vorteilhaft, wenn das Hüllrohr verliersicher an dem Betätigungsstab gehalten ist.

Die Verliersicherung ist, nach einer Ausführungsform, dadurch gebildet, dass ein im Wesentlichen in Radialrichtung federbeaufschlagtes, in eine umlaufende Nut mit Axialspiel eingreifendes Rastelement vorhanden ist, wobei das Axialspiel dem Verfahrweg zwischen der Arbeits- und Ruhestellung entspricht. Ein Handhabungswerkzeug nach der vorgenannten Ausführungsform weist einen Betätigungsstab und ein Hüllrohr auf, welche aneinander lösbar fixiert sind. Das Hüllrohr weist gegenüber dem Betätigungsstab einen durch das Axialspiel der umlaufenden Nut definierten Verfahrweg auf. Gleichzeitig dient die umlaufende Nut der Verliersicherung des Hüllrohres gegenüber dem Betätigungsstab und erfüllt somit besonders vorteilhaft eine Doppelfunktion.

Die Verliersicherung des Hüllrohres gegenüber dem Betätigungsstab kann, gemäß einer Ausführungsform, dadurch verbessert werden, dass sich am Außenumfang des Betätigungsstabes, zwischen dem vorderen Ende und der umlaufenden Nut, wenigstens ein herausragender Ringvorsprung befindet. Sollte sich das Hüllrohr aus der umlaufenden Nut mit Axialspiel lösen, so ist dieses zusätzlich durch den umlaufenden Ringvorsprung gesichert.

Zur drehfesten Verbindung zwischen Hüllrohr und Betätigungsstab weist das Handhabungswerkzeug, nach einer weiteren Ausführungsform, eine Drehfixierung auf. Diese besteht aus einer Abflachung und einem Vorsprung, wobei sich die Abflachung an dem Betätigungsstab wenigstens über den vorderen Teilbereich axial erstreckt und bis in die Stirnfläche hinein reicht. Auf der Innenseite des Hüllrohres ist ein Vorsprung vorhanden, der mit einer Flachseite an der Abflachung anliegt. Der Kraftschluss einer solchen drehfesten Verbindung zwischen Hüllrohr und Betätigungsstab wird im vorderen Bereich des Handhabungswerkzeuges hergestellt. Es ergibt sich daraus der Vorteil, dass beim Einführen des Betätigungsstabes in das Hüllrohr der passgenaue Sitz, der zu der drehfesten Verbindung führt, also der Sitz zwischen der Flachseite des Vorsprunges und der Abflachung, zunächst nicht hergestellt werden muss. Es muss zunächst lediglich der Betätigungsstab in das Hüllrohr eingeführt werden. Der zu der drehfesten Verbindung führende Formschluss zwischen einer Flachseite des Hüllrohres und einer Abflachung des Betätigungsstabes wird erst dann hergestellt, wenn der Betätigungsstab bereits verliersicher in dem Hüllrohr gehalten ist. Die Bediensicherheit des Handhabungswerkzeuges kann signifikant verbessert werden.

Die Abflachung, welche sich über einen Teilbereich des Betätigungsstabes erstreckt, ist, gemäß einer Weiterbildung, von dem Boden einer Axialnut gebildet. Der sich in diese Axialnut hinein erstreckende Vorsprung ist komplementär ausgebildet. Der formschlüssig in die Axialnut hineingreifende Vorsprung, insbesondere dessen senkrecht zum Boden der Axialnut orientierten Wandteile bilden klar definierte Anschlagflächen mit den ebenfalls vorzugsweise senkrecht zu dem Boden orientierten Seitenwänden der Axialnut aus. Vorteilhaft kann auf diese Weise eine zuverlässige, mechanisch hoch belastbare und drehfeste Verbindung zwischen dem Hüllrohr und dem Betätigungsstab erreicht werden.

Nach einer Ausführungsform ragt der Vorsprung über die Stirnseite des Hüllrohres hinaus, und bildet zumindest ein Arretierelement aus. Vorteilhaft erfüllt dieser zumindest eine Vorsprung eine Doppelfunktion. Der Vorsprung wirkt sowohl als Arretierelement zur Drehfixierung des Implantates gegenüber dem Handhabungswerkzeug als auch als Teil der Drehfixierung zwischen dem Hüllrohr und dem Betätigungsstab.

Gemäß einer Ausführungsform sind die von der Mittellängsachse wegweisenden Außenkanten der Sperrbacken so ausgestaltet, dass diese jeweils den gleichen Radialabstand zu einem die Sperrbacken umschreibenden Umkreis aufweisen. Die in konstantem Abstand zu einem Umkreis angeordneten Außenkanten der Sperrbacken dienen einer Reduzierung des Radialspiels zwischen dem Fixierelement des Handhabungswerkzeuges und den implantatseitigen Anschlagflächen des Gegenelementes.

Der radiale Formschluss zwischen den Sperrbacken und den Anschlagflächen des Gegenelementes kann dadurch verbessert werden, dass die Außenkanten der Sperrbacken als Segmente eines gemeinsamen Kreisbogens weitergebildet werden. Die mechanische Verbindung zwischen Implantat und Handhabungswerkzeug wird weiter verbessert, indem die zu dem hinteren Ende des Handhabungswerkzeuges gerichteten Seiten der Sperrbacken, die mit den entsprechenden Flächen des Gegenelementes zusammen wirken, eine Neigung gegenüber der Mittellängsachse des Betätigungsstabes aufweisen. Nach einer solchen Ausführungsform schließen die nach hinten gerichteten Seiten der Sperrbacken mit der Mittellängsachse des Betätigungsstabes einen sich zum vorderen Ende des Handhabungswerkzeuges öffnenden, spitzen Winkel ein. Derart geneigte Flächen der Sperrbacken bewirken eine Zentrierung des Fixierelementes in dem implantatseitigen Gegenelement um die Mittellängsachse des Betätigungsstabes.

Die vorgenannten Flächen des Fixierelementes sind, gemäß einer weiteren Ausführungsform, Teilflächen einer sich zum hinteren Ende des Handhabungswerkzeuges verjüngenden Konusfläche. Die derart ausgestalteten Sperrbacken liegen großflächig an der entsprechenden implantatseitigen Gegenfläche an, und erlauben daher eine Aufnahme des Implantates durch das Handhabungswerkzeug mit einer geringen Flächenpressung.

Der Betätigungsstab, einschließlich des Griffteils, ist, nach einer weiteren Ausführungsform, von einer zentralen Bohrung durchsetzt, die der Aufnahme einer Antriebswelle dient. Die Antriebswelle weist an ihrem vorderen Ende ein Antriebsritzel und an ihrem hinteren Ende einen Drehknopf auf. Durch eine Führung der Antriebswelle im Inneren des Betätigungsstabes kann das Verletzungsrisiko für das umliegende Gewebe bei Betätigung der Antriebswelle minimiert werden.

Nach einer weiteren Ausführungsform ist die Antriebswelle zwischen einer Leerlaufposition und einer Eingriffsposition axial verschiebbar. In der Leerlaufposition liegt die Stirnseite des Antriebsritzels axial hinter, und in der Eingriffsposition axial vor der Stirnseite des Betätigungsstabes. Befindet sich die Antriebswelle in der Eingriffsposition kann beispielsweise die Höhe eines Implantates verändert werden. Um sicherzustellen, dass während einer Positionsveränderung des Implantates dessen Höhe unverändert bleibt, kann die Antriebswelle von der Eingriffsposition in die Leerlaufposition verbracht werden. Die Bedienung des Handhabungswerkzeuges kann auf diese Weise deutlich verbessert werden.

Gemäß einer Weiterbildung weist das Griffteil an seinem hinteren Ende eine radial umlaufende Nut auf. Ein in dem Drehgriff vorhandenes, federbeaufschlagtes Rastelement greift mit einem in Axialrichtung wirksamen Formschluss in diese Nut ein. Besonders vorteilhaft erfüllt die Nut eine Doppelfunktion. Sie wirkt gemeinsam mit dem Rastelement zum einen als Verliersicherung, und zum anderen ist durch den in Axialrichtung wirksamen Formschluss zwischen Rastelement und Nut die Leerlaufposition der Antriebswelle definiert.

Die Eingriffsposition der Antriebswelle ist durch eine an dem Griffteil vorhandene, mit dem Drehknopf zusammenwirkende Anschlagfläche definiert. Vorteilhaft muss beim Lösen der Antriebswelle aus der Eingriffsposition keine Verrastung überwunden werden, da die Eingriffsposition mechanisch mit Hilfe einer Anschlagfläche definiert ist. Die Antriebswelle ist also leicht von der Eingriffsposition in die Leerlaufposition zurückziehbar.

Ein erfindungsgemäßes Implantat weist einen Körper und einen an diesem vorhandenes Gegenelement zur lösbaren Fixierung eines Handhabungswerkzeuges auf. Das Gegenelement weist zumindest zwei bezüglich einer Achse des Körpers beabstandete Hinterschnitte mit jeweils einer in Richtung des Innenraums des Körpers orientierten Haltefläche auf. Das Gegenelement weist außerdem, zur Drehfixierung des Implantats gegenüber einem Handhabungswerkzeug, eine nicht koaxial zur der Achse orientierte Anschlagfläche auf. Das erfindungsgemäße Implantat erlaubt eine besonders einfache Verrastung mit einem Handhabungswerkzeug durch einen formschlüssigen Hintergriff an den in Richtung des Innenraums des Körpers orientieren Halteflächen. Eine Drehfixierung zwischen einem Handhabungswerkzeug und einer koaxial zu der Achse des Implantates orientierten Fläche ist - vorausgesetzt das Handhabungswerkzeug wird entlang der Achse des Implantates mit diesem verbunden - lediglich reibschlüssig möglich. Die Drehfixierung zwischen Implantat und Handhabungswerkzeug ist also auf die maximale Reibungskraft des Reibschlusses limitiert. Eine Drehfixierung mit Hilfe einer nicht koaxial zur der Achse des Implantates orientierten Anschlagfläche erlaubt einen verbesserten Kraftschluss für die Drehfixierung des Implantates.

Vorteilhafte Ausgestaltungen des erfindungsgemäßen Implantates gehen aus den von Anspruch 20 abhängigen Unteransprüchen hervor. Dabei kann das erfindungsgemäße Implantat mit den Merkmalen eines, insbesondere denen mehrere Unteransprüche kombiniert werden.

Nach einer ersten Ausführungsform umfasst die Anschlagfläche eine parallel zu der Achse verlaufende Fläche. Ist die Anschlagfläche parallel zur der Achse des Implantates orientiert, so wird das Einfügen des Handhabungswerkzeuges entlang der Achse des Implantates wesentlich vereinfacht.

Nach einer weiteren Ausführungsform weist das Implantat solche Halteflächen auf, die jeweils von der Wandung einer in den Körper eingelassenen Ausnehmung gebildet sind. Durch die Integration der Halteflächen in die Wandung kann die Oberfläche des Implantates weitgehend glatt gehalten werden, was das Verletzungsrisiko beim Einsetzen des Implantates verringert.

Gemäß einer weiteren Ausführungsform schließen die Halteflächen mit der Achse des Körpers einen sich zu dem Innenraum des Körpers hin öffnenden spitzen Winkel ein. Derart gegenüber der Achse des Körpers geneigte Halteflächen bewirken eine axiale Zentrierung des Implantates gegenüber seiner Achse. Eine solche Maßnahme erlaubt eine spielfreie Aufnahme des Implantates durch ein Handhabungswerkzeug.

Zur weiteren Verbesserung der Aufnahme des Implantates durch ein Handhabungswerkzeug sind die Halteflächen als Teilflächen einer sich zu seinem Innenraum hin aufweitenden Konusfläche weitergebildet. Durch eine derartige Ausgestaltung der Halteflächen liegen diese großflächig an den entsprechenden Gegenflächen eines Handhabungswerkzeuges an, was zu einer geringen Flächenpressung an den Halteflächen führt und eine sichere Aufnahme des Implantates durch ein Handhabungswerkzeug gestattet.

Bei dem erfindungsgemäßen Implantat kann es sich, gemäß einer Ausführungsform, um ein höhenverstellbares Implantat handeln. Dieses ist durch die folgenden Merkmale gekennzeichnet: Der Körper des Implantates ist durch ein erstes und ein zweites Bauteil gebildet, die längs einer Mittellängsachse eines Implantates gegeneinander axial beweglich sind und drehfest aneinander gehalten sind. Die Bauteile weisen jeweils zumindest zwei an einer Basis fixierte Wandsegmente auf, die sich in Richtung der Mittellängsachse erstrecken und jeweils einen Radialabstand zu dieser aufweisen. Jeweils zwei in Umfangsrichtung benachbarte Wandsegmente flankieren einen Zwischenraum, in den sich ein Wandsegment des jeweils anderen Bauteils erstreckt und darin axial geführt ist. In dem von den Wandsegmenten umschlossenen Innenraum ist ein Antriebselement angeordnet, welches mit dem zweiten Bauteil nach der Art eines Schraubengetriebes zusammenwirkt. Das Antriebselement weist einen koaxial zu der Mittellängsachse angeordneten Zahnkranz auf, der zur Drehbetätigung des Antriebselementes dient. In Lastrichtung stützt sich das Antriebselement an dem ersten Bauteil ab. Zumindest ein Wandsegment des ersten Bauteils weist eine dieses durchsetzende Zugriffsöffnung auf, über die der Zahnkranz zur Drehbetätigung des Antriebselementes mit Hilfe eines Handhabungswerkzeuges zugänglich ist.

Ein Implantat nach der vorgenannten Ausführungsform ist durch das in seinem Innenraum vorhandene Antriebselement höhenverstellbar. Die Höhenverstellung erfolgt durch ein Antriebselement, welches mit Hilfe eines Handhabungswerkzeuges angetrieben wird. Folglich ist es sowohl möglich das Implantat mit einem Handhabungswerkzeug sicher und mechanisch zuverlässig aufzunehmen, als auch dieses in seiner Höhe zu verstellen. Die Manipulation des Implantates erfolgt also sowohl, was die Verstellung der Höhe als auch seine Positionierung anbelangt, lediglich mit einem einzigen Handhabungswerkzeug. Die Handhabung des Implantates kann auf diese Weise wesentlich vereinfacht werden.

Weitere vorteilhafte Ausgestaltungen des erfindungsgemäßen Handhabungswerkzeug, Implantates sowie des erfindungsgemäßen Teilesatzes gehen aus den vorstehend nicht angesprochenen Unteransprüchen, sowie aus der nachfolgend erläuterten Zeichnung hervor.

### Dabei zeigen deren

- Fig. 1a bis c: ein Handhabungswerkzeug und Detailansichten von dessen vorderem Ende in Perspektivansicht und im Querschnitt,
- Fig. 2 und 4: ein Handhabungswerkzeug in Perspektivansicht,
- Fig. 3a bis c: einen Betätigungsstab, ein Hüllrohr und eine Antriebswelle für ein Handhabungswerkzeug in Perspektivansicht,
- Fig. 5a bis c: perspektivische Detailansichten des vorderen Endes eines Handhabungswerkzeuges,
- Fig. 6a bis b: Details eines Handhabungswerkzeuges im Bereich der Verbindung zwischen dem Betätigungsstab und dem Hüllrohr in Perspektivansicht,
- Fig. 7: einen perspektivischen Längsschnitt durch ein Handhabungswerkzeug im Bereich seines hinteren Endes,
- Fig. 8: ein Implantat in Perspektivansicht und
- Fig. 9 und 10: ein Implantat sowie das vordere Ende eines zugehörigen Handhabungswerkzeuges in Perspektivansicht bzw. im Querschnitt.

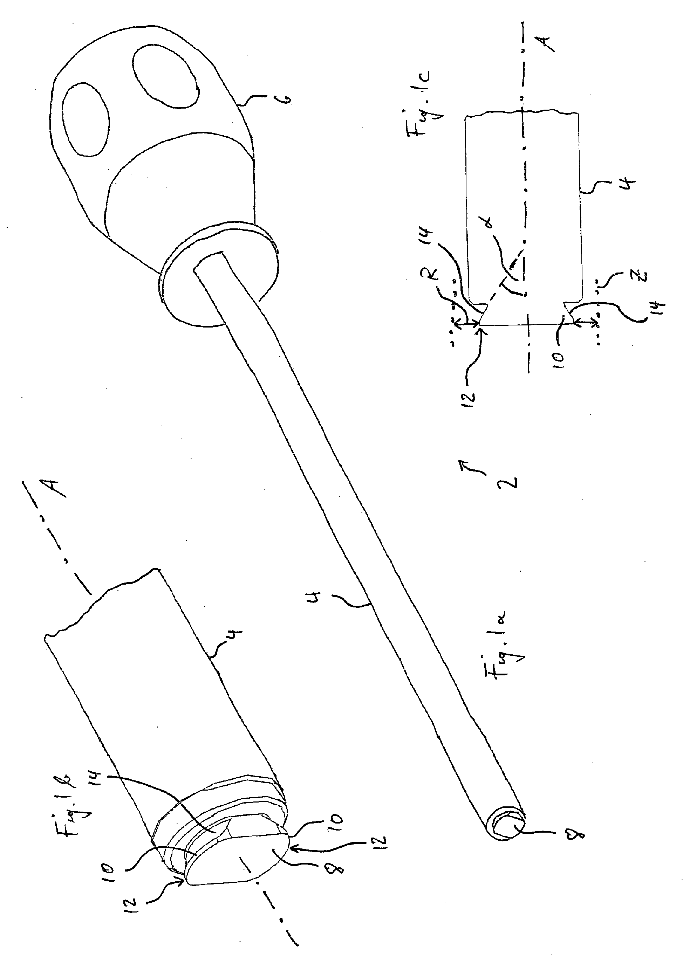

Fig. 1a zeigt ein Handhabungswerkzeug 2 mit einem Betätigungsstab 4, an dessen hinterem Ende sich ein Griffteil 6 und an dessen vorderem Ende sich ein Fixierelement 8 befindet. Das Handhabungswerkzeug 2 dient zum Einsetzen eines medizinischen Implantates. Das Fixierelement 8 weist, wie die Detailansicht des vorderen Endes des Betätigungsstabes 4 in Fig. 1b zeigt, zwei Sperrbacken 10 auf, die dem Hintergriff von entsprechenden implantatsseitigen Gegenelementen dienen. Die Sperrbacken 10 sind bezüglich eines gedachten Zylinders, der sich koaxial zu der Mittellängsachse A des Handhabungswerkzeuges 2 erstreckt, in Radialrichtung beabstandet. Gemäß der in Fig. 1b gezeigten Ausführungsform sind die Sperrbacken 10 des Fixierelementes 8 bezüglich der Mittellängsachse A einander diametral gegenüberliegend angeordnet. Die Außenkanten 12 der Sperrbacken 10 beschreiben Segmente eines gemeinsamen Kreisbogens. Insbesondere beschreiben die Außenkanten 12 der Sperrbacken 10 Segmente eines um die Mittellängsachse A gezogenen Kreisbogens. Fig. 1c zeigt einen Querschnitt durch das vordere Ende des Betätigungsstabes 4. Die Außenkanten 12 der Sperrbacken 10 weisen einen gemeinsamen Abstand R zu einem gedachten Zylinder Z auf, der sich um die Mittellängsachse A herum erstreckt.

Bei den Sperrbacken 10 des Handhabungswerkzeuges 2 kann es sich abweichend von den Darstellungen in den Fig. 1a bis c um andersartig geformte Körper handeln. Beispielsweise können die Sperrbacken 10 durch von der Mittellängsachse A wegweisende Stäbe oder Quader gebildet sein. Außerdem ist es nicht erforderlich, dass die Außenkanten 12 der Sperrbacken 10, wie in Fig. 1b dargestellt, einem gemeinsamen Kreisbogen um die Mittellängsachse A folgen. Beispielsweise können solche Sperrbacken 10 eingesetzt werden, welche als Außenkanten 12 einander bezüglich der Mittellängsachse A gegenüberliegende parallele Kanten aufweisen.

Das in den Figuren 1a bis c gezeigte Handhabungswerkzeug 2 dient der Aufnahme eines medizinischen Implantates. Zur Verriegelung des Handhabungswerkzeuges 2 mit dem medizinischen Implantat weisen die Sperrbacken 10 am vorderen Ende des Betätigungsstabes 4, wie die Figuren 1b und 1c zeigen, Flächen 14 auf, welche mit den entsprechenden implantatsseitigen Gegenflächen zusammenwirken. In Fig. 1c ist die Richtung einer der Flächen 14 in gestrichelter Linie bis in den Bereich der Mittellängsachse A verlängert dargestellt. Die Fläche 14 ist so orientiert, dass diese mit der Mittellängsachse A einen sich in Richtung der Vorderseite des Handhabungswerkzeuges 2 öffnenden spitzen Winkel α einschließt. Die Sperrbacken 10 können abweichend von der Darstellung in den Figuren 1a bis 1c derart ausgestaltet sein, dass diese lediglich Teilflächen aufweisen, welche so orientiert sind, dass sie mit der Mittellängsachse A einen spitzen Winkel α einschließen.

Die in den Figuren 1b und 1c gezeigten Flächen 14 sind so ausgestaltet, dass sie Teil einer sich zum hinteren Ende des Handhabungswerkzeuges 2 verjüngenden Konusfläche sind. Wie anhand von Fig. 1b deutlich wird, liegen die Flächen 14 der Sperrbacken 10 auf einer gemeinsamen Kegelfläche, deren Kegelspitze auf der Mittellängsachse A liegt. Der Kegelmantel erstreckt sich symmetrisch um die Mittellängsachse A herum.

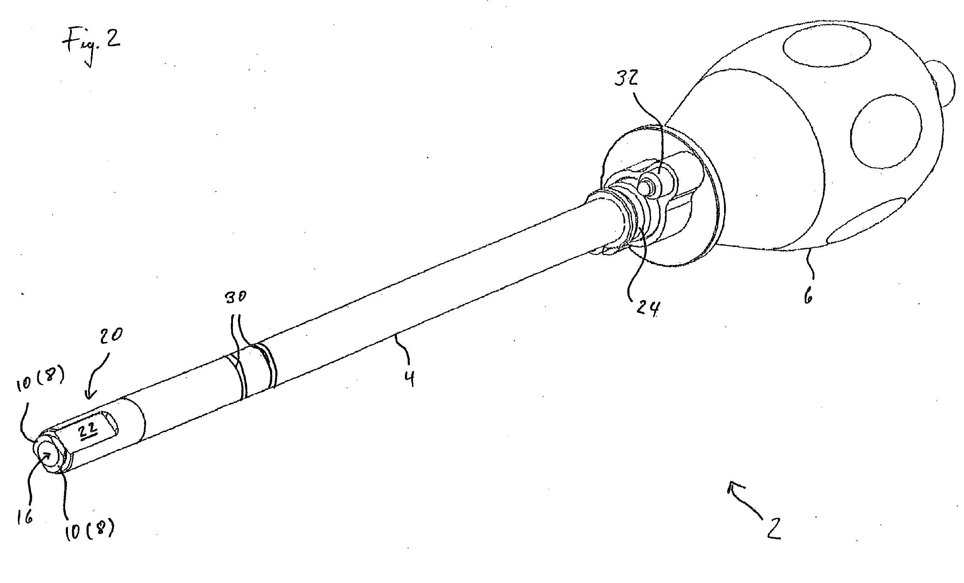

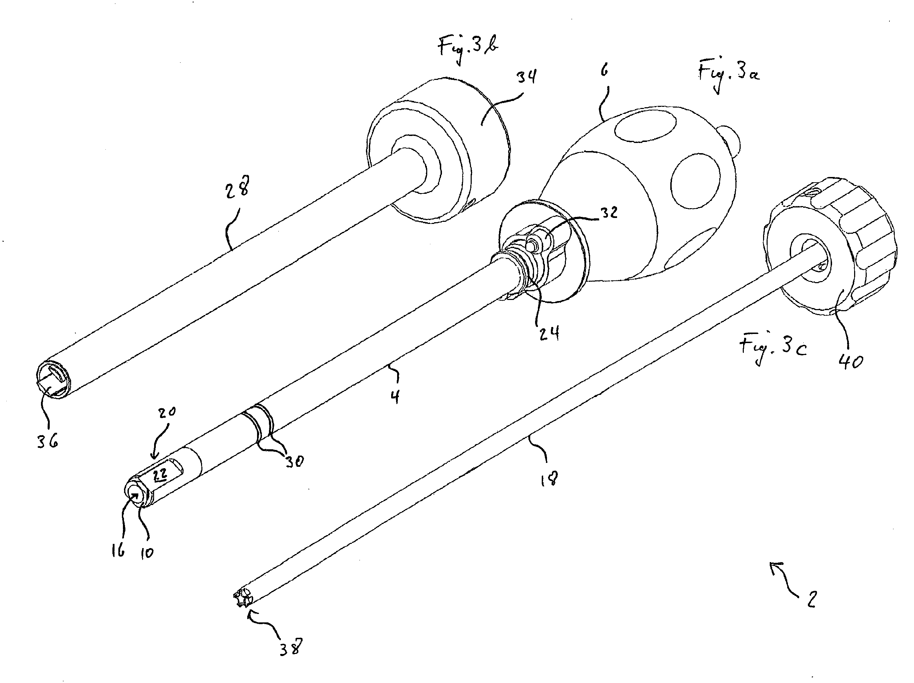

Fig. 2 zeigt ein weiteres Ausführungsbeispiel für ein Handhabungswerkzeug 2, dieses weist an seinem vorderen Ende ein Fixierelement 8 mit Sperrbacken 10 auf. Das Handhabungswerkzeug 2 ist in Längsrichtung entlang der Mittellängsachse A, die in Fig. 2 aus Gründen der Übersichtlichkeit nicht dargestellt ist, von einer zentralen Bohrung 16 durchsetzt. Die Bohrung 16 dient der Aufnahme einer Antriebswelle 18, die im Zusammenhang mit den Figuren 3a bis c beschrieben werden soll. Die Sperrbacken 10 des in Fig. 2 gezeigten Handhabungswerkzeuges 2 sind so ausgestaltet, wie bereits im Zusammenhang mit den Figuren 1a bis c beschrieben.

Der Betätigungsstab 4 weist an seinem vorderen Ende eine Axialnut 20 auf, die sich bis in die Stirnseite des Betätigungsstabes 4 erstreckt. Die Axialnut 20 ist in die Wandung des Betätigungsstabes 4 eingelassen. Ihr Boden ist von der Form einer Abflachung 22, die sich an diese anschließenden Wandflächen erstrecken sich senkrecht zum Boden der Axialnut 20. Im Querschnitt betrachtet, ist die Axialnut 20 also u-förmig. Die Axialnut 20 ist Teil einer Einrichtung zur Dreharretierung des medizinischen Implantates an dem Handhabungswerkzeug 2, worauf im Zusammenhang mit den Fig. 3a bis c näher eingegangen werden soll.

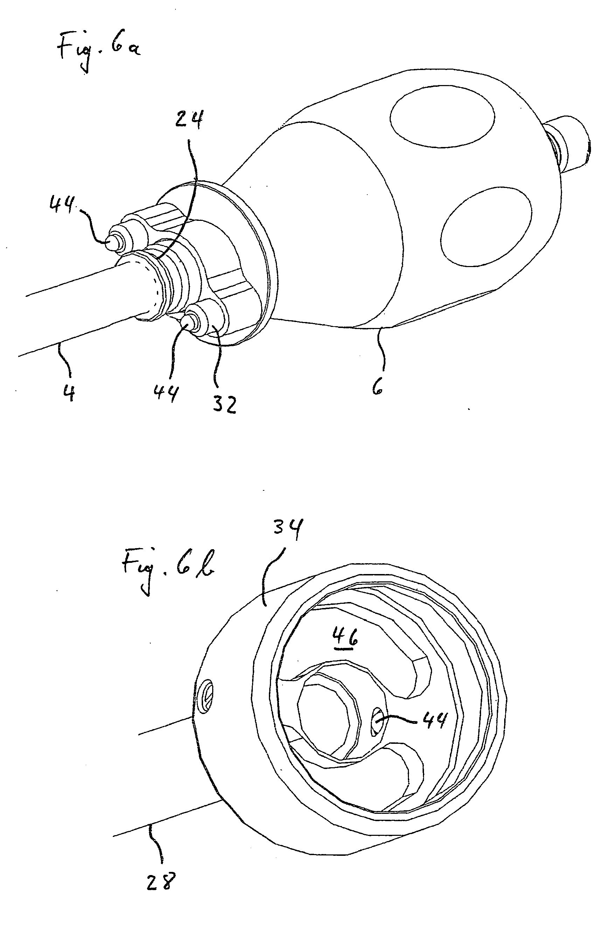

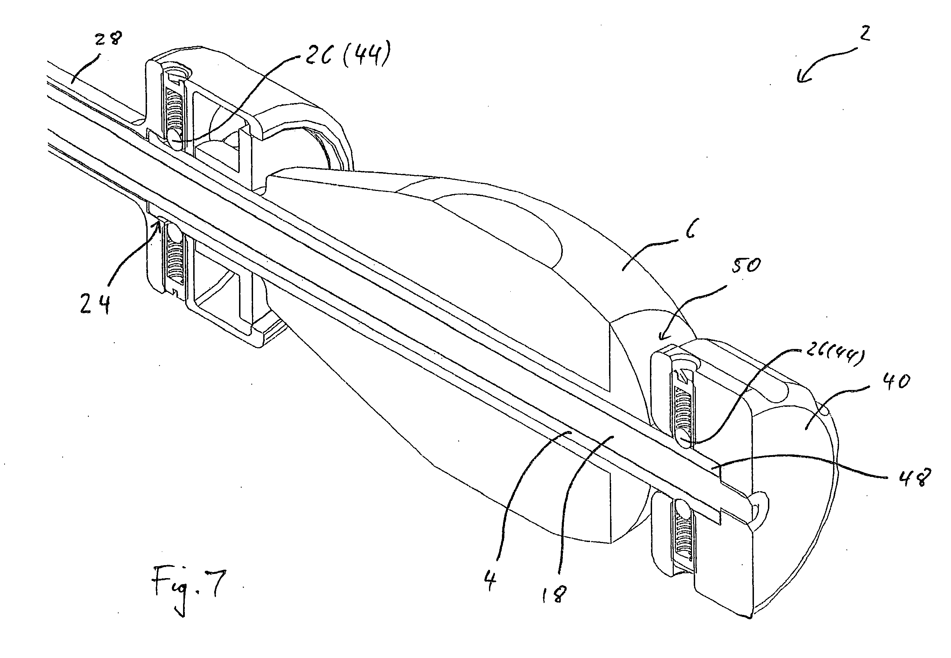

Der Betätigungsstab 4 des Handhabungswerkzeuges 2 weist an seinem hinteren Ende eine umlaufende Nut 24 zum Eingriff eines federbelasteten Rastelementes auf. Die radial umlaufende Nut 24 dient der Verliersicherung eines Hüllrohres 28, in welches der Betätigungsstab 4 eingeschoben werden kann. Die Verliersicherung zwischen dem in Fig. 3b dargestellten Hüllrohr 28 und dem Betätigungsstab 4 wird im Zusammenhang mit den Figuren 6 und 7 näher erläutert. Die zwischen dem vorderen Ende des Betätigungsstabes 4 und der umlaufenden Nut 24 vorhandenen Ringvorsprünge 30 dienen ebenfalls der Verliersicherung des Hüllrohres 28. Der Betätigungsstab 4 wird aus zwei einzeln gefertigten Hälften zusammengesetzt, die vorzugsweise durch Laserschweißen miteinander verbunden werden. Die entstehende Schweißnaht bildet die Ringvorsprünge 30 aus.

Im Bereich des Griffteiles 6 befinden sich an dem Betätigungsstab 4 Federelemente 32, von denen in der in Fig. 2 gewählten perspektivischen Ansicht lediglich eines sichtbar ist. Die Federelemente 32 dienen der mechanischen Vorspannung des Hüllrohres 28 gegenüber dem Betätigungsstab 4, worauf näher im Zusammenhang mit den Figuren 6a und b eingegangen werden soll.

Ein Handhabungswerkzeug 2 zur Aufnahme eines medizinischen Implantates erfüllt mehrere Aufgaben. Zum einen dient es der mechanisch zuverlässigen und einfach lösbaren Halterung eines medizinischen Implantates. Das Implantat ist dabei in axialer Richtung des Handhabungswerkzeuges 2 durch die Backen 10 fixiert. Fig. 3a zeigt den bereits aus Fig. 2 bekannten Teil des Handhabungswerkzeuges 2, der im Wesentlichen den Betätigungsstab 4 und damit verbundene Bauteile umfasst. Das Handhabungswerkzeug 2 dient jedoch nicht nur der axialen Fixierung, sondern erlaubt auch eine drehfeste Verbindung des Implantates mit dem Handhabungswerkzeug 2.

Fig. 3b zeigt das bereits angesprochene Hüllrohr 28, in das der Betätigungsstab 4 eingeführt werden kann. Das Hüllrohr 28 ist an seinem hinteren Ende zu einem Gehäuse 34 erweitert. An seinem vorderen Ende weist das Hüllrohr 28 zwei einander gegenüberliegende Vorsprünge 36 auf, die aus der Stirnseite des Hüllrohres 28 herausragen, und ein Arretierelement zur drehfesten Aufnahme eines medizinischen Implantates ausbilden. Die Innenseiten der Vorsprünge 36 bilden planparallele Flächen bezüglich der aus Gründen der Übersichtlichkeit nicht dargestellten Mittellängsachse A des Hüllrohres 28 aus. Die Vorsprünge 36 erstrecken sich auf der Innenseite des Hüllrohres 28 in dessen vorderem Bereich. Die Vorsprünge 36 sind passgenau zu den am vorderen Ende des Betätigungsstabes 4 vorhandenen Axialnuten 20 ausgebildet. Zur Drehfixierung des Hüllrohres 28 gegenüber dem Betätigungsstab 4 greifen die Vorsprünge 36 in die Axialnuten 20 des Betätigungsstabes 4 ein, wobei die innenliegenden Flachseiten der Vorsprünge 36 an den Abflachungen 22 der Axialnuten 20 anliegen. Die Drehfixierung des Hüllrohres 28 gegenüber dem Betätigungsstab 4 wird außerdem dadurch erreicht, dass die Axialnut 20 in Fig. 3a nicht näher dargestellte, im Wesentlichen senkrecht zur der Abflachung 22 orientierte Anschlagflächen aufweist, die mit den entsprechenden Gegenflächen der Vorsprünge 36 zusammen wirken. Bei den Anschlagflächen handelt es sich um die senkrecht zum Boden der Abflachung 22 orientierten Wandflächen (vgl. Fig. 2).

Die Axialnuten 20 des Betätigungsstabes 4 sowie die Vorsprünge 36 des Hüllrohres 28 erstrecken sich in deren vorderen Bereichen der jeweiligen Bauteile. Dies erleichtert das Einführen des Betätigungsstabes 4 in das Hüllrohr 28. So wird der Betätigungsstab 4 von dem hinteren Ende her, also im Bereich des Gehäuses 34, in das Hüllrohr 28 eingeführt, wobei das Hüllrohr 28 und der Betätigungsstab 4 zu diesem Zeitpunkt noch nicht drehrichtig zueinander orientiert sein müssen. Erst wenn der Betätigungsstab 4 weiter in das Hüllrohr 28 eingeschoben wird, müssen die beiden Bauteile zum Eingriff der Vorsprünge 36 in die Axialnuten 20 drehrichtig zueinander orientiert sein. Das Hüllrohr 28 ist nunmehr mit dem Betätigungsstab 4, insbesondere aber auch mit dem Griffteil 6, drehfest verbunden. Die Vorsprünge 36 dienen außerdem dem Eingriff in entsprechende implantatseitige Gegenelemente, so dass auf diese Weise ein medizinisches Implantat drehfest mit dem Handhabungswerkzeug 2 verbunden werden kann.

Das medizinische Implantat kann weitere interne mechanische Funktionen aufweisen, beispielsweise kann das Implantat höhenverstellbar sein. Um beispielsweise ein in dem Implantat vorhandenes Getriebe antreiben zu können, weist der in Fig. 3a gezeigte Betätigungsstab 4 eine zentrale Bohrung 16 auf, in welcher eine Antriebswelle 18, die in Fig. 3c dargestellt ist, geführt werden kann. Die Antriebswelle 18 weist an ihrem vorderen Ende ein Antriebsritzel 38 auf. An ihrem hinteren Ende ist ein Drehknopf 40 mit der Antriebswelle 18 verbunden. Die Antriebswelle 18 wird in die zentrale Bohrung 16 des Betätigungsstabes 4 eingeführt, wobei ihre Länge so gewählt ist, dass das Antriebsritzel 38 im zusammengesetzten Zustand aus der Stirnfläche des Betätigungsstabes 4 hervortritt. Diese Position ist dazu geeignet ein innerhalb des Implantates vorhandenes Antriebselement zu betätigen. Aus diesem Grund soll diese Position der Antriebswelle 18 bzw. des Antriebsritzels 38 als Eingriffsposition bezeichnet werden. Die Antriebswelle 18 ist außerdem in eine Leerlaufposition zurückziehbar, in welcher das Antriebsritzel 38 nicht mit einem gegebenenfalls vorhandenen Antriebselement des aufgenommenen medizinischen Implantates zusammenwirkt. In der Leerlaufposition tritt das Antriebsritzel axial hinter die Stirnseite des Betätigungsstabes 4 zurück. Hinsichtlich weiterer Ausführungen wird auf Fig. 7 verwiesen.

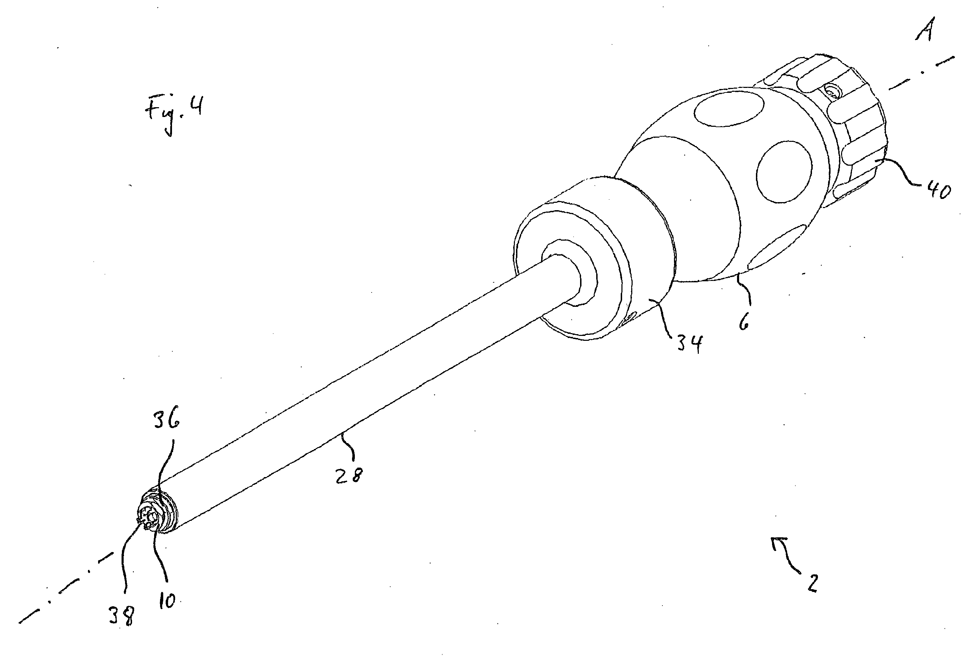

Fig. 4 zeigt ein, aus den in den Fig. 3a bis 3c gezeigten Einzelteilen, zusammengesetztes Handhabungswerkzeug 2. Die Sperrbacken 10 am vorderen Ende des Handhabungswerkzeuges 2 sind in Umfangsrichtung voneinander beabstandet. In den Zwischenräumen kommen die Vorsprünge 36 zu liegen. Entlang der Mittellängsachse A erstreckt sich die Antriebswelle 18, die an ihrem vorderen Ende zu einem Antriebsritzel 38 ausgebildet ist, welches axial gesehen vor die Stirnseite der Sperrbacken 10 tritt. Die Vorsprünge 36, welche der drehfesten Aufnahme eines medizinischen Implantates dienen, sind mit dem Hüllrohr 28, welches den Betätigungsstab 4 und die Antriebswelle 18 umschließt, vorzugsweise einstückig ausgebildet. Das gesamte Hüllrohr 28 ist in axiale Richtung verschiebbar. Zu diesem Zweck kann das Hüllrohr 28 an dem Gehäuse 34 zurückgezogen werden. Durch die Lage von Griffteil 6 und Gehäuse 34 ist eine einhändige Bedienung ermöglicht. Der am hinteren Ende des Handhabungswerkzeuges 2 vorhandene Drehknopf 40 dient der Drehbetätigung des Antriebsritzels 38.

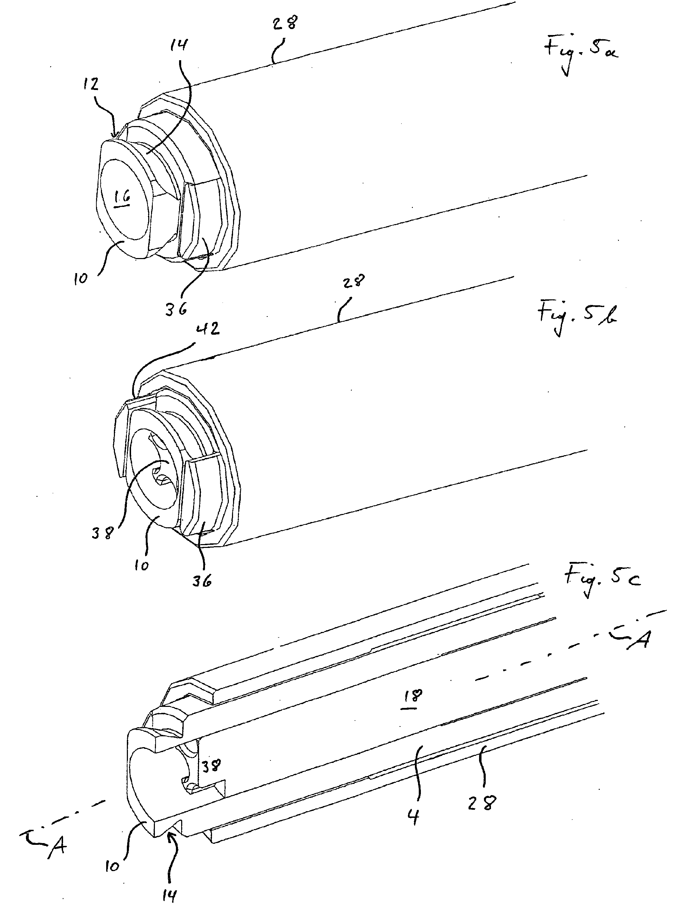

Die Figuren 5a bis 5c zeigen jeweils das vordere Ende des Handhabungswerkzeuges 2. Sichtbar ist in diesem Fall lediglich das außenliegende, vordere Ende des Hüllrohres 28. Die Verriegelung eines medizinischen Implantates mit dem Handhabungswerkzeug 2, in Axialrichtung des Handhabungswerkzeuges, erfolgt über die Flächen 14, die in Wechselwirkung mit den entsprechenden implantatseitigen Gegenflächen treten.

Fig. 5a zeigt die Stellung des Handhabungswerkzeuges 2, in welcher, durch eine 90°-Drehung um die Mittellängsachse A, das Handhabungswerkzeug 2 mit einem Implantat verriegelt werden kann. Die mit dem Hüllrohr 28 verbundenen Vorsprünge 36 sind soweit zurück gezogen, dass die zu dem hinteren Ende des Handhabungswerkzeuges 2 zeigenden Flächen 14 der Sperrbacken 10 bei einer Drehung um die Mittellängsachse A ungehindert in entsprechend geformte Hinterschnitte eines Implantates eingreifen können. Zur spielfreien Aufnahme des Implantates gleiten dabei vorzugsweise die kreissegmentförmigen Außenkanten 12 der Sperrbacken 10 in entsprechend geformten Fugen des Implantates.

Fig. 5c zeigt den in Fig. 5a gezeigten vorderen Teil des Handhabungswerkzeuges 2 in einer geschnittenen Perspektivansicht. Die Antriebswelle 18 einschließlich des Antriebsritzels 38 ist soweit in das Innere des Betätigungsstabes 4 zurück gezogen, dass eine ungehinderte Verrastung der Sperrbacken 10 mit einem Implantat erfolgen kann.

Fig. 5b zeigt den vorderen Teil des Handhabungswerkzeuges 2, wobei die mit dem Hüllrohr 28 verbundenen Vorsprünge 36 zur drehfesten Verbindung des Handhabungswerkzeuges 2 mit einem medizinischen Implantat axial vorgeschoben sind. Die Stirnseiten der Sperrbacken 10 und der Vorsprünge 36 liegen nun in einer gemeinsamen Ebene. Die drehfeste Verbindung zwischen dem Implantat und dem Handhabungswerkzeug 2 wird vornehmlich durch die senkrecht zur der Stirnseite der Sperrbacken 10 orientierte Fixierflächen 42 erreicht, die sich jeweils an den axial orientierten Seitenkanten der Vorsprünge 36 erstrecken.

Das hintere Ende des Betätigungsstabes 4 ist in Fig. 6a gezeigt, Fig. 6b zeigt dasjenige des Hüllrohres 28. Das Hüllrohr 28 ist an seinem hinteren Ende zu dem Gehäuse 34 erweitert. Innerhalb des Gehäuses 34 befinden sich Ausnehmungen 46, in welche die an dem hinteren Ende des Betätigungsstabes 4 vorhandenen Federelemente 32 eingreifen. Die Federelemente 32 sind dabei nach der Art von Kugeldruckstücken ausgestaltet. Ein am vorderen Ende der Federelemente 32 vorhandene Kugel 44 liegt am Boden der Ausnehmung 46 an, wenn das Hüllrohr 28 auf den Betätigungsstab 4 vollständig aufgeschoben wird. Das Federelement 32 stützt sich also mit seinem hinteren Ende an dem Betätigungsstab 4 und mit seinem vorderen Ende an dem Hüllrohr 28, bzw. dem Gehäuse 34 ab.

Am hinteren Ende des Betätigungsstabes 2 befindet sich eine radial umlaufende Nut 24. In dem Innenraum des Gehäuses 34 befinden sich die federbelasteten Kugeln 44 zweier Kugeldruckstücke, von denen lediglich eines sichtbar wird. Diese greifen als Rastelemente mit Axialspiel in die radial umlaufende Nut 24 ein. Das Hüllrohr 28 ist durch diesen Kraftschluss in Axialrichtung mit dem Betätigungsstab 4 lösbar verbunden. Gleichzeitig bewirkt das Zusammenspiel zwischen den in dem Gehäuse 34 vorhandenen Kugeldruckstücken mit der axial umlaufenden Nut 24 eine Verliersicherung des Hüllrohres 28 gegenüber dem Betätigungsstab 4. Wie bereits im Zusammenhang mit den Figuren 2 und 3a erwähnt, weist der Betätigungsstab 4 zwischen seinem vorderen Ende und der radial umlaufenden Nut 4 Ringvorsprünge 30 auf. Die in dem Gehäuse 34 vorhandenen Kugeldruckstücke wirken mit den Ringvorsprüngen 30 des Betätigungsstabes 4 als zusätzliche Verliersicherung.

Wie bereits im Zusammenhang mit Fig. 4 erwähnt, kann das Hüllrohr 28 an seinem Gehäuse 34 gegen die Kraft der Federelemente 32 soweit zurück gezogen werden, dass eine ungehinderte Verrastung mit einem Implantat ermöglicht ist. Die Verrastung des Handhabungswerkzeuges 2 mit dem Implantat erfolgt anschließend durch Wirkung der Federkraft der Federelemente 32, sobald sich die Vorsprünge 36 in der passenden Position zum Eingriff in entsprechende Gegenelemente des Implantates befinden. Somit erfolgt die Drehsicherung des Implantates gegenüber dem Handhabungswerkzeug 2 nach erfolgter axialer Fixierung mit Hilfe der Sperrbacken 10 automatisch und kann somit nicht vergessen werden.

Fig. 7 zeigt das hintere Ende des Handhabungswerkzeuges 2. Das in die umlaufende Nut 24 eingreifende Rastelement 26 des Hüllrohres 28 von der Form einer Kugel 44 weist in der umlaufenden Nut 24 des Betätigungsstabes 4 ein Axialspiel auf. Dieses Axialspiel definiert die in den Figuren 5a und 5b gezeigten Positionen des Hüllrohres 28. Die mit dem Hüllrohr 28 verbundenen Vorsprünge 36 können somit um einem definierten axialen Verfahrweg bewegt werden.

Der Griffteil 6 des Betätigungsstabes 4 weist an seinem hinteren Ende eine weitere radial umlaufende Nut auf, in die ein weiteres Rastelement 26 mit einem in Axialrichtung wirksamen Formschluss eingreift. Das Rastelement 26 ist in den Drehgriff 40 eingelassen, und kann wiederum von der Form einer Kugel 44 eines Kugeldruckstückes sein. Der Griffteil 6 ist an seinem hinteren Ende um einen Fortsatz 48 verlängert, welcher die radial umlaufende Nut (nicht näher bezeichnet) trägt.

Wie bereits im Zusammenhang mit Fig. 4 erwähnt, weist die Antriebswelle 18 bzw. das mit ihr verbundene Antriebsritzel 38 eine Leerlauf- und eine Arbeitsposition auf. In der Leerlaufposition ist, wie in Fig. 7 dargestellt, die Antriebswelle 18 durch einen axial wirksamen Formschluss lösbar fixiert. Diese Fixierung dient gleichzeitig der Verliersicherung der Antriebswelle 18 gegenüber dem Betätigungsstab 4 bzw. dem Griffteil 6. In einer Eingriffsposition wird die Antriebswelle 18 an dem Drehknopf 40 in axialer Richtung des Betätigungsstabes 4 nach vorn geschoben. Zur eindeutigen Definition der Eingriffsposition des Antriebsritzels 38 weist der Griffteil 6 an seinem hinteren Ende eine Anschlagfläche 50 auf, die mit einer entsprechend gegenüberliegenden Anschlagfläche des Drehknopfes 40 zusammenwirkt. Die Eingriffsposition ist durch den Kontakt der Anschlagsflächen 50 definiert, es ist jedoch keine in axialer Richtung wirksame Verrastung hergestellt. Das Fehlen dieser Verrastung bewirkt, dass die Antriebswelle 18 aus der Eingriffsposition sehr leicht in die stabilere Leerlaufposition zurückgezogen werden kann.

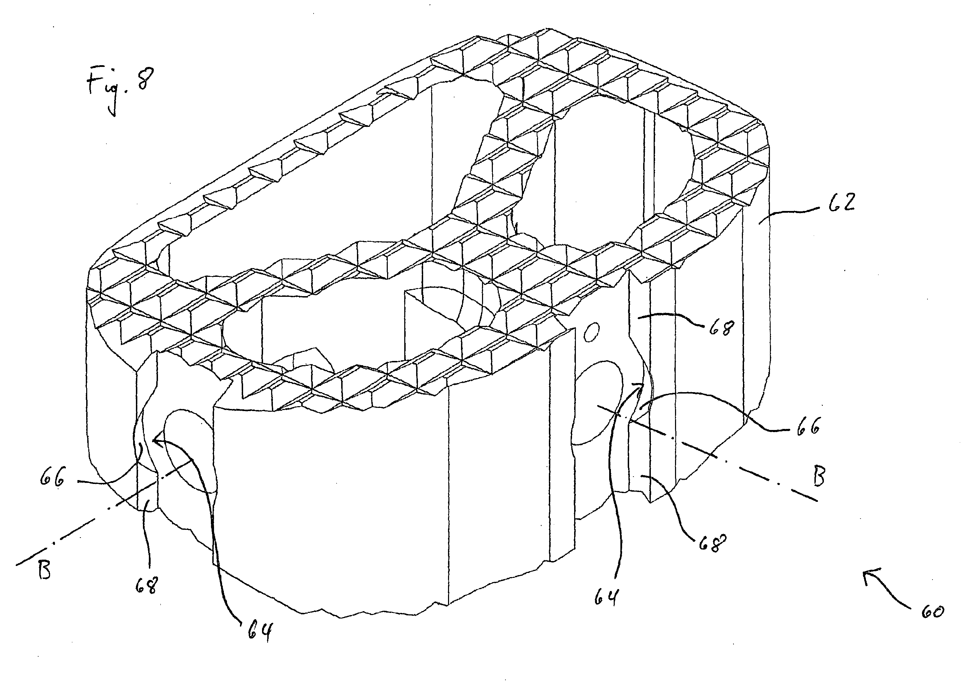

Fig. 8 zeigt ein Implantat 60 nach einem Ausführungsbeispiel. Das Implantat 60 weist einen Körper 62 auf, an dem ein Gegenelement zur lösbaren Fixierung eines Handhabungswerkzeuges 2, insbesondere dem zuvor beschriebenen Handhabungswerkzeug 2, vorhanden ist. Der Körper 62 des Implantates 60 weist zwei Achsen B auf. Ein Handhabungswerkzeug wird koaxial zu diesen Achsen B mit dem Implantat 60 verbunden.

Zur kraftschlüssigen Verbindung des Implantates 60 mit einem Handhabungswerkzeug 2 weist das Implantat 60 ein Gegenelement mit zumindest zwei bezüglich seiner Achsen B beabstandeten Hinterschnitten 64 auf. Jeder Hinterschnitt 64 weist eine in Richtung des Innenraums des Körpers 62 orientierte Haltefläche 66 auf.

Zur Handhabung des in Fig. 8 gezeigten Implantates 60 mit dem Handhabungswerkzeug 2 greift dieses mit den Sperrbacken 10 in die Hinterschnitte 64. Es liegt nunmehr eine kraftschlüssige Verbindung zwischen den Flächen 14 der Sperrbacken 10 und denen in Richtung des Innenraums des Körpers 62 orientierten Halteflächen 66 der Hinterschnitte 64 vor.

Die Hinterschnitte 64 sind in die Wandung des Körpers 62 des Implantates 60 eingelassen, können jedoch beispielsweise auch durch auf der Oberfläche des Körpers 62 vorhandene Leisten oder ähnlich geformte Körper gebildet sein.

Das Implantat 60 weist Anschlagflächen 68 auf, die mit den Fixierflächen 42 (vgl. Fig. 5b) des Vorsprungs 36 des Hüllrohres 28 zusammenwirken. Das Implantat 60 kann auf diese Weise drehfest mit dem Handhabungswerkzeug 2 verbunden werden. Die Anschlagflächen 68 sind parallel zu der jeweiligen Achse B orientiert, was das Einführen des Handhabungswerkzeuges 2 wesentlich vereinfacht.

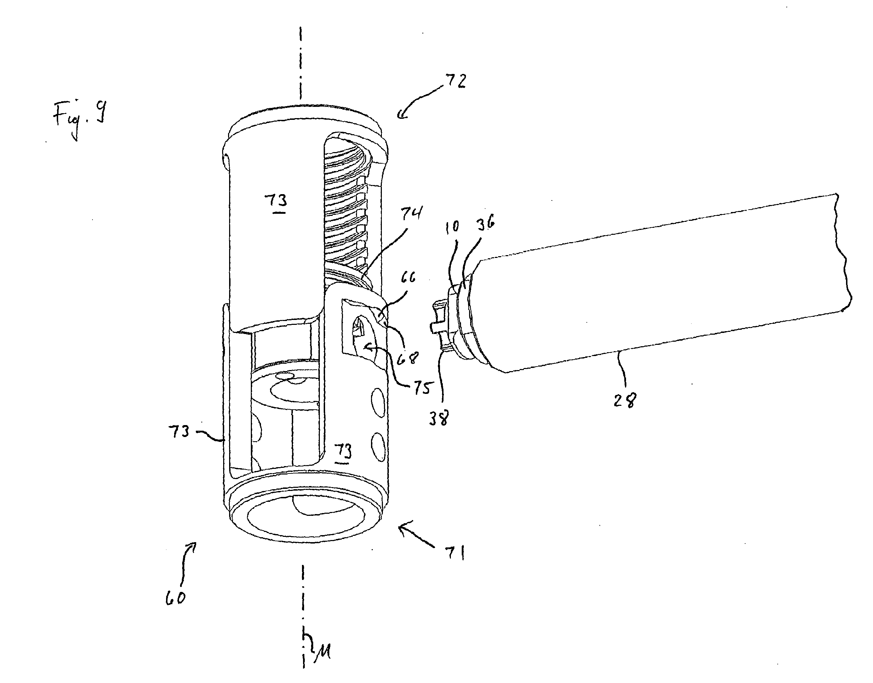

Fig. 9 zeigt ein weiteres Ausführungsbeispiel für ein Implantat 60 gemeinsam mit dem vorderen Teil eines Handhabungswerkzeuges 2. Das Implantat 60 und das Handhabungswerkzeug 2 bilden einen vorteilhaft zusammenwirkenden Teilesatz aus.

Das in Fig. 9 gezeigte Implantat 60 weist ein erstes und ein zweites Bauteil 71, 72 auf, die den Körper 62 des Implantates 60 bilden. Das erste und zweite Bauteil 71, 72 sind entlang einer Mittellängsachse M des Implantates 60 axial beweglich und gegeneinander drehfest gehalten. Die beiden Bauteile 71, 72 weisen jeweils zumindest zwei an einer Basis fixierte Wandsegmente 73 auf. Die Wandsegmente 73 erstrecken sich jeweils in einem Radialabstand sowohl in Richtung der Mittellängsachse M als auch in Umfangsrichtung des Implantates 60. Einander benachbarte Wandsegmente 73 flankieren in Umfangsrichtung einen Zwischenraum, in welchen sich jeweils ein Wandsegment 73 des anderen Bauteils 71, 72 erstreckt und darin axial geführt ist.

Die beiden Bauteile 71, 72 sind gegeneinander verschiebbar, so dass die Höhe bzw. Länge des Implantates 60 verstellbar ist. Zur Verstellung der Länge bzw. Höhe des Implantates 60 wirkt ein im Innenraum vorhandenes Antriebselement 74 mit dem zweiten Bauteil 72 nach der Art eines Schraubengetriebes zusammen. Zu diesem Zweck weist das Antriebselement 74 ein Außengewinde auf, welches mit einem auf der Innenseite der Wandsegmente 73 des zweiten Bauteils 72 vorhandenen Innengewinde kämmt. Durch Drehen des Antriebselementes 74 um die Mittellängsachse M des Implantates 60 kann das zweite Bauteil 72 gegenüber dem ersten Bauteil 71 verfahren werden. Zur Betätigung des Antriebelementes 74 befindet sich in einem Wandsegment 73 eine Zugriffsöffnung 75, durch welche das Antriebsritzel 38 des Handhabungswerkzeuges 2 auf das Antriebselement 74 zugreifen kann.

Das Antriebselement 74 weist auf seiner Unterseite ein für das Antriebsritzel 38 (welches durch die Zugriffsöffnung 75 in den Innenraum des Implantates 60 verbracht wird) zugängliches Kronenrad auf. Das Kronenrad des Antriebselementes 74 und das Antriebsritzel 38 wirken nach der Art eines Kronenradgetriebes zusammen. Das Antriebselement 74 ist gegenüber dem ersten Bauteil 71 in Lastrichtung, also entlang der Mittellängsachse M des Implantates 60, abgestützt.

Um eine Positionierung des Implantates 60 zu ermöglichen, ist das Handhabungswerkzeug 2 mit dem Implantat 60 über die Sperrbacken 10 in axialer Richtung und mit den Vorsprüngen 36 drehfest verbunden.

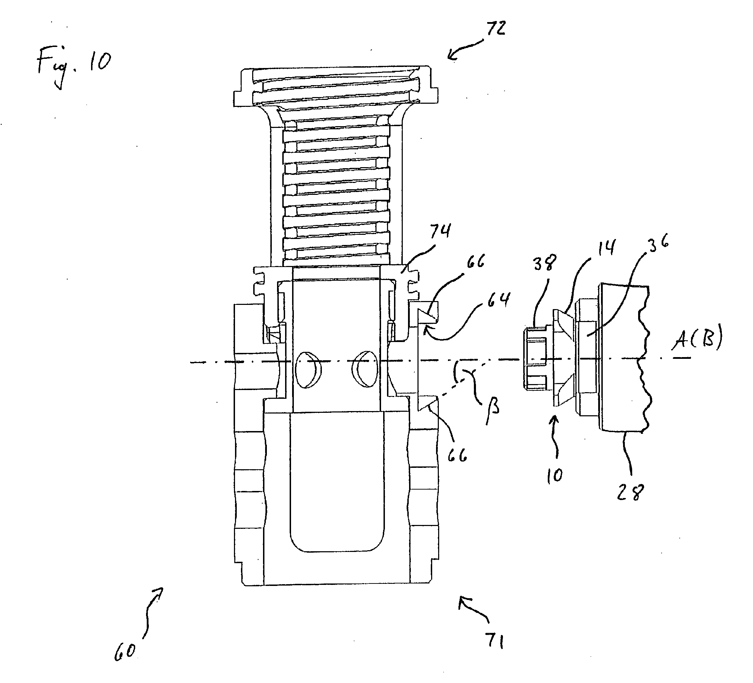

Fig. 10 zeigt einen Querschnitt durch das aus Fig. 9 bekannte Implantat 60 und den vorderen Teil des Handhabungswerkzeuges 2, in einer Ebene, die die Mittellängsachse A als auch eine Achse B des Implantates 60 enthält. Das Handhabungswerkzeug 2 wird so mit dem Implantat 60 verbunden, dass die Mittellängsachse A koinzident zu der Achse B des Implantates 60 liegt.

Die Halteflächen 66 schließen mit der Achse B einen zu dem Innenraum des Implantates 60 hin geöffneten spitzen Winkel β ein. Insbesondere kann der Winkel β dem weiter oben erwähnten Winkel α entsprechen, den die Sperrbacken 10 mit der Mittellängsachse A in Richtung des vorderen Teils des Handhabungswerkzeuges 2 einschließen. Zur Verbesserung der kraftschlüssigen Verbindung zwischen den Halteflächen 66 des Implantates 60 und den Flächen 14 der Sperrbacken 10 des Handhabungswerkzeuges 2, sind die Halteflächen 66 Teil einer sich zu dem Innenraum des Implantates 60 hin aufweitenden Konusfläche.

Das Implantat 60 weist nicht koaxial zur der Achse B orientierte Anschlagflächen 68 auf (vgl. auch Fig. 8 und 9). Vorzugsweise sind die Anschlagflächen 68 parallel zu der Achse B orientiert. Die Anschlagflächen 68 wirken mit den Fixierflächen 42 des Handhabungswerkzeuges 2 zusammen (vgl. Fig. 5b), und ermöglichen die Drehfixierung des Implantates 60 gegenüber dem Handhabungswerkzeug 2.

Die Verbindung zwischen dem Handhabungswerkzeug 2 und dem Implantat 60 wird dadurch hergestellt, dass zunächst der Betätigungsstab 4 bzw. die Sperrbacken 10 um 90° gegenüber der in den Figuren 9 und 10 gezeigten Position um die Mittellängsachse A gedreht wird bzw. werden. Die Sperrbacken 10 werden anschließend in dem Bereich der auf der Außenseite des Implantates 60 vorhandenen Abplattung verbracht. Dabei wird das Handhabungswerkzeug 2 so geführt, dass seine Mittellängsachse A und die Achse B des Implantates 60 koinzidieren. Anschließend wird das Handhabungswerkzeug 2 um 90° um seine Mittellängsachse A gedreht, so dass die Sperrbacken 10 in die Hinterschnitte 64 eingreifen. Hierbei kommen die Flächen 14 der Sperrbacken 10 und die Halteflächen 66 des Implantates 60 formschlüssig aufeinander zu liegen.

Das Implantat 60 und das Handhabungswerkzeug 2 sind nunmehr axial, also entlang der Mittellängsachse A des Handhabungswerkzeuges 2 bzw. entlang der Achse B des Implantates 60, miteinander verbunden. Eine Drehfixierung des Implantates 60 ist bereits zu diesem Zeitpunkt durch einen gegebenenfalls vorhandenen Reibschluss zwischen der Fläche 14 der Sperrbacken 10 und der Haltefläche 66 des Implantates 60 möglich.

Durch Eingreifen der Vorsprünge 36 an den Anschlagflächen 68 kann nunmehr das Implantat 60 gegenüber dem Handhabungswerkzeug 2 drehfest verriegelt werden. Zur Höhenverstellung des Implantates 60 kann anschließend das Antriebsritzel 38 entlang der Mittellängsachse A des Handhabungswerkzeuges 2 soweit nach vorn geschoben werden, dass dieses seine Eingriffsposition erreicht, und mit dem auf der Unterseite des Antriebselementes 74 vorhandenen Kronenrad zum Antrieb des Antriebselementes kämmt.

## Patentansprüche

1. Handhabungswerkzeug (2) für ein medizinisches Implantat mit einem Betätigungsstab (4), an dessen hinterem Ende ein Griffteil (6) und an dessen vorderem Ende ein Fixierelement (8) zur lösbaren axialen Fixierung des Implantats vorhanden ist, das zumindest zwei einen Radialabstand zur Mittellängsachse (A) des Betätigungsstabes (4) und - bezüglich eines sich koaxial zur Mittellängsachse (A) erstreckenden gedachten Zylindermantels (Z) - einen gegenseitigen Abstand in Umfangsrichtung aufweisende, Sperrbacken (10) aufweist
**gekennzeichnet durch**
eine Einrichtung zur Dreharretierung des Handhabungswerkzeugs (2) an einem Implantat, welche wenigstens ein drehfest mit dem Betätigungsstab (4) verbundenes, mit einer Anschlagfläche (68) des Implantats drehfest zusammenwirkendes Arretierelement umfasst, das zwischen einer vorderen Position, seiner Arbeitsstellung, und einer hinteren Position, seiner Ruhestellung, axial verschiebbar an dem Betätigungsstab (4) gehalten ist, sowie **dadurch gekennzeichnet, dass** die Sperrbacken jeweils zum Hintergreifen wenigstens eines implantatseitigen Gegenelements dienen.

2. Handhabungswerkzeug (2) nach Anspruch 1,
**dadurch gekennzeichnet,**
**dass** das wenigstens eine Arretierelement in Richtung seiner Arbeitsstellung federbelastet ist.

3. Handhabungswerkzeug (2) nach Anspruch 1 oder 2,
**dadurch gekennzeichnet,**
**dass** die Einrichtung zur Dreharretierung ein den Betätigungsstab (4) umschließendes und axial an diesem verschiebbares Hüllrohr (28) umfasst, dessen vorderes Ende wenigstens ein fest damit verbundenes Arretierelement trägt.

4. Handhabungswerkzeug (2) nach Anspruch 3,
**gekennzeichnet durch**,
wenigstens ein Federelement (32), welches sich mit seinem hinteren Ende am Betätigungsstab (4) und seinem vorderen Ende am Hüllrohr (28) abstützt.

5. Handhabungswerkzeug (2) nach Anspruch 4,
**dadurch gekennzeichnet,**
**dass** das hintere Ende des Hüllrohrs (28) unter Ausbildung eines Gehäuses (34) radial erweitert ist, wobei das wenigstens eine Federelement (32) in dem Gehäuse (34) angeordnet ist.

6. Handhabungswerkzeug (2) nach einem der Ansprüche 3 bis 5,
**dadurch gekennzeichnet,**
**dass** das Hüllrohr (28) verliersicher an dem Betätigungsstab (4) gehalten ist.

7. Handhabungswerkzeug (2) nach Anspruch 6,
**dadurch gekennzeichnet,**
**dass** die Verliersicherung durch ein im Wesentlichen in Radialrichtung federbeaufschlagtes, in eine umlaufende Nut (24) mit Axialspiel eingreifendes Rastelement (26) gebildet ist, wobei das Axialspiel dem Verfahrweg zwischen der Arbeits- und Ruhestellung entspricht.

8. Handhabungswerkzeug (2) nach Anspruch 7,
**dadurch gekennzeichnet,**
**dass** am Außenumfang des Betätigungsstabes (4), zwischen dem vorderen Ende und der umlaufenden Nut (24) wenigstens ein herausragender Ringvorsprung (30) vorhanden ist.

9. Handhabungswerkzeug (2) nach einem der Ansprüche 3 bis 8,
**gekennzeichnet durch**,
eine Drehfixierung aus einer Abflachung und einem Vorsprung, wobei sich die Abflachung (22) an dem Betätigungsstab (4) wenigstens über den vorderen Teilbereich axial erstreckt, und bis in seine Stirnfläche hineinreicht, und der Vorsprung (36) auf der Innenseite des Hüllrohres (28) vorhanden ist, und mit einer Flachseite an der Abflachung (22) anliegt.

10. Handhabungswerkzeug (2) nach Anspruch 9,
**dadurch gekennzeichnet,**
**dass** die Abflachung (22) von dem Boden einer Axialnut (20) gebildet ist, und der sich in diese hineinerstreckende Vorsprung (36) komplementär ausgebildet ist.

11. Handhabungswerkzeug (2) nach Anspruch 10,
**dadurch gekennzeichnet,**
**dass** der zumindest eine Vorsprung (36) über die Stirnseite des Hüllrohres (28) hinausragt und zumindest ein Arretierelement ausbildet.

12. Handhabungswerkzeug (2) nach einem der vorstehenden Ansprüche,
**dadurch gekennzeichnet,**
**dass** die Sperrbacken (10) von der Mittellängsachse (A) wegweisende, jeweils den gleichen Radialabstand (R) zu einem die Sperrbacken (10) umschreibenden Umkreis aufweisende Außenkanten (12) besitzen.

13. Handhabungswerkzeug (2) nach Anspruch 12,
**dadurch gekennzeichnet,**
**dass** die Außenkanten (12) der Sperrbacken (10) Segmente eines gemeinsamen Kreisbogens beschreiben.

14. Handhabungswerkzeug (2) nach Anspruch 12 oder 13,
**dadurch gekennzeichnet,**
**dass** an den zum hinteren Ende des Handhabungswerkzeugs (2) weisenden Seiten der Sperrbacken (10) mit einem Gegenelement zusammenwirkende Flächen (14) vorhanden sind, die mit der Mittellängsachse (A) des Betätigungsstabes (4) einen sich zu dessen vorderem Ende öffnenden Winkel (α) einschließen.

15. Handhabungswerkzeug (2) nach Anspruch 14,
**dadurch gekennzeichnet,**
**dass** die Flächen (14) sich zum hinteren Ende hin verjüngende Konusflächen sind.

16. Handhabungswerkzeug (2) nach einem der vorstehenden Ansprüche,
**dadurch gekennzeichnet,**
**dass** der Betätigungsstab (4) einschließlich des Griffteils (6) von einer zentralen Bohrung (16) zur Aufnahme einer Antriebswelle (18) durchsetzt ist, die an ihrem vorderen Ende ein Antriebsritzel (38) und an ihrem hinteren Ende einen Drehknopf (40) aufweist.

17. Handhabungswerkzeug (2) nach Anspruch 16,
**dadurch gekennzeichnet,**
**dass** die Antriebswelle (18) zwischen einer Leerlaufposition und einer Eingriffsposition axial verschiebbar ist, wobei in der Leerlaufposition die Stirnseite des Antriebsritzels (38) axial hinter, und in der Eingriffsposition axial vor der Stirnseite des Betätigungsstabes (4) liegt.

18. Handhabungswerkzeug (2) nach einem der Ansprüche 16 oder 17,
**dadurch gekennzeichnet,**
**dass** das Griffteil (6) an seinem hinteren Ende eine radial umlaufende Nut aufweist, in die ein in dem Drehknopf (40) vorhandenes, federbeaufschlagtes Rastelement mit einem in Axialrichtung wirksamen Formschluss eingreift.

19. Handhabungswerkzeug (2) nach Anspruch 17 oder 18,
**dadurch gekennzeichnet,**
**dass** die Eingriffsposition durch eine an dem Griffteil (6) vorhandene, mit dem Drehknopf (40) zusammenwirkende Anschlagfläche (50) definiert ist.

20. Medizinisches Implantat (60) mit einem eine Achse (B) aufweisenden Körper (62) und einem an diesem vorhandenen Gegenelement zur lösbaren Fixierung eines Handhabungswerkzeuges, wobei das Gegenelement, zur Drehfixierung des Implantates (60) gegenüber einem Handhabungswerkzeug (2), eine nicht koaxial zu der Achse (B) orientierte Anschlagfläche (68) aufweist,
**dadurch gekennzeichnet,**
**dass** das Gegenelement mindestens zwei bezüglich der Achse (B) beabstandete Hinterschnitte (64) mit jeweils einer in Richtung eines Innenraums des Körpers (62) orientierten Haltefläche (66) aufweist.

21. Medizinisches Implantat (60) nach Anspruch 20,
**dadurch gekennzeichnet,**
**dass** die Anschlagfläche (68) eine parallel zu der Achse (B) verlaufende Fläche umfasst.

22. Medizinisches Implantat (60) nach Anspruch 20 oder 21,
**dadurch gekennzeichnet,**
**dass** die Halteflächen (66) jeweils von der Wandung einer in den Körper (62) eingelassenen Ausnehmung gebildet sind.

23. Medizinisches Implantat (60) nach einem der Ansprüche 20 bis 22,
**dadurch gekennzeichnet,**
**dass** die Halteflächen (66) mit der Achse (B) des Körpers (62) einen sich zu dessen Innenraum hin öffnenden spitzen Winkel (β) einschließen.

24. Medizinisches Implantat (60) nach Anspruch 23,
**dadurch gekennzeichnet,**
**dass** die Halteflächen (66) Teilflächen einer sich zu dem Innenraum hin aufweitenden Konusfläche sind.

25. Medizinisches Implantat (60) nach einem der Ansprüche 20 bis 24,
**dadurch gekennzeichnet, dass**
- der Körper (62) durch ein erstes und ein zweite Bauteil (71,72) gebildet ist, die längs einer Mittellängsachse (M) des Implantates (60) gegeneinander drehfest und axial beweglich aneinander gehalten sind, wobei die Bauteile (71,72) jeweils zumindest zwei an einer Basis fixierte Wandsegmente (73) aufweisen, die sich in Richtung der Mittellängsachse (M) erstrecken und jeweils einen Radialabstand zu dieser aufweisen, und jeweils zwei in Umfangsrichtung benachbarte Wandsegmente (73) einen Zwischenraum
- flankieren, in den sich ein Wandsegment (73) des anderen Bauteils (71,72) erstreckt und darin axial geführt ist, ein Antriebselement (74) in dem von den Wandsegmenten (73) umschlossenen Innenraum angeordnet ist, welches mit dem zweiten Bauteil (72) nach der Art eines Schraubengetriebes zusammenwirkt, sich in Lastrichtung an dem ersten Bauteil (71) abstützt und einen koaxial zur Mittellängsachse (11) angeordneten Zahnkranz aufweist, der zu seiner Drehbetätigung dient,
wobei
- zumindest ein Wandsegment (73) des ersten Bauteils (72) eine dieses durchsetzende Zugriffsöffnung (75) aufweist, über die der Zahnkranz zur Drehbetätigung des Antriebselementes (74) mit Hilfe eines Handhabungswerkzeuges (2) zugänglich ist.

26. Teilesatz für die Implantationsmedizin mit einem Handhabungswerkzeug (2) nach einem der Ansprüche 1 bis 19 und einem medizinischen Implantat (60) nach einem der Ansprüche 20 bis 25.

## Claims

1. Manipulating tool (2) for a medical implant, said tool comprising an actuating rod (4) having a grip part (6) at the rear end thereof and a fixing element (8) at the front end thereof for releasably axially fixing the implant, said fixing element having at least two clamping jaws (10) arranged at a radial distance from the central longitudinal axis (A) of the actuating rod (4) and, relative to an imaginary cylinder surface (Z) extending coaxially with said central longitudinal axis (A), arranged in a mutually spaced apart manner in a peripheral direction, **characterised by** a device for rotationally locking the manipulating tool (2) to an implant, said device comprising at least one locking element which is connected in a rotationally engaged manner to the actuating rod (4), cooperates in a rotationally fixed manner with a bearing surface (68) of the implant, and is held axially displaceably on the actuating rod (4) between a front position, defining an operating position thereof, and a rear position, defining a rest position thereof, and **characterized in that** the clamping jaws are each used to engage, from behind, at least one counter element on the implant side.

2. Manipulating tool (2) according to claim 1, **characterised in that** the at least one locking element is spring-loaded in the direction of the operating position thereof.

3. Manipulating tool (2) according to claim 1 or 2, **characterised in that** the device for rotational locking comprises a sheathing tube (28) enclosing the actuating rod (4) and being axially displaceable thereon, the front end of said sheathing tube carrying at least one locking element connected rigidly thereto.

4. Manipulating tool (2) according to claim 3, **characterised by** at least one spring element (32), which is supported by its rear end on the actuating rod (4) and by its front end on the sheathing tube (28).

5. Manipulating tool (2) according to claim 4, **characterised in that** the rear end of the sheathing tube (28) is radially widened, forming a housing (34), the at least one spring element (32) being arranged in the housing (34).

6. Manipulating tool (2) according to one of claims 3 to 5, **characterised in that** the sheathing tube (28) is mounted captively on the actuating rod (4).

7. Manipulating tool (2) according to claim 6, **characterised in that** the sheathing tube is mounted captively by a latching element (26) which is spring-loaded substantially in the radial direction and engages in a peripheral groove (24) with axial play, the axial play corresponding to the path of movement between the operating position and the rest position.

8. Manipulating tool (2) according to claim 7, **characterised in that** at least one projecting annular protrusion (30) is provided on the outer periphery of the actuating rod (4), between said front end and the peripheral groove (24).

9. Manipulating tool (2) according to one of claims 3 to 8, **characterised by** a rotational fixing formed of a flattened portion and a protrusion, the flattened portion (22) extending axially on the actuating rod (4), at least over the front sub-region, and reaching into a front face thereof, and the protrusion (36) being provided on the inner face of the sheathing tube (28) and bearing against the flattened portion (22) by means of a flat face.

10. Manipulating tool (2) according to claim 9, **characterised in that** the flattened portion (22) is formed by the base of an axial groove (20), and the protrusion (36) extending therein is formed in a complementary manner.

11. Manipulating tool (2) according to claim 10, **characterised in that** the at least one protrusion (36) projects beyond the front face of the sheathing tube (28) and forms at least one locking element.

12. Manipulating tool (2) according to one of the preceding claims, **characterised in that** the clamping jaws (10) each have outer edges (12) facing away from the central longitudinal axis (A) and arranged at the same radial distance (R) from a circle circumscribing the clamping jaws (10).

13. Manipulating tool (2) according to claim 12, **characterised in that** the outer edges (12) of the clamping jaws (10) describe segments of a common circular arc.

14. Manipulating tool (2) according to claim 12 or 13, **characterised in that** surfaces (14) cooperating with a counter element are provided on the sides of the clamping jaws (10) facing towards the rear end of the manipulating tool (2), said surfaces, together with the central longitudinal axis (A) of the actuating rod (4), enclosing an angle (α) opening towards the front end of the actuating rod.

15. Manipulating tool (2) according to claim 14, **characterised in that** the surfaces (14) are conical surfaces tapering towards the rear end.

16. Manipulating tool (2) according to one of the preceding claims, **characterised in that** the actuating rod (4), including the grip part (6), is penetrated by a central bore (16) for receiving a drive shaft (18), which has a drive pinion (38) at its front end and a rotary knob (40) at its rear end.

17. Manipulating tool (2) according to claim 16, **characterised in that** the drive shaft (18) is axially displaceable between a neutral position and an engagement position, the front face of the drive pinion (38) being located axially behind the front face of the actuating rod (4) in the neutral position and being located axially in front of the front face of the actuating rod (4) in the engagement position.

18. Manipulating tool (2) according to one of claims 16 or 17, **characterised in that** the grip part (6) has a radial peripheral groove in its rear end, a spring-loaded latching element provided in the rotary knob (40) engaging in said groove with a positive fit effective in the axial direction.

19. Manipulating tool (2) according to claim 17 or 18, **characterised in that** the engagement position is defined by a bearing surface (50) provided on the grip part (6) and cooperating with the rotary knob (40).

20. Medical implant (60), comprising a body (62) having an axis (B) and a counter element provided on said body for releasably fixing a manipulating tool thereto, said counter element having a bearing surface (68) oriented non-coaxially with the axis (B) for rotationally fixing the implant (60) relative to a manipulating tool (2), **characterised in that** the counter element has at least two undercuts (64) spaced apart relative to the axis (B) and each having a holding surface (66) oriented in the direction of an interior of the body (62).

21. Medical implant (60) according to claim 20, **characterised in that** the bearing surface (68) comprises a surface extending parallel to the axis (B).

22. Medical implant (60) according to claim 20 or 21, **characterised in that** the holding surfaces (66) are each formed by the wall of a recess formed in the body (62).

23. Medical implant (60) according to one of claims 20 to 22, **characterised in that** the holding surfaces (66), together with the axis (B) of the body (62), enclose an acute angle (β) opening towards the interior of said body.

24. Medical implant (60) according to claim 23, **characterised in that** the holding surfaces (66) are sub-surfaces of a conical surface widening towards the interior.

25. Medical implant (60) according to one of claims 20 to 24, **characterised in that**
- the body (62) is formed by a first and a second component (71, 72), which are held along a central longitudinal axis (M) of the implant (60) so as to be rotationally fixed relative to one another and axially movably against one another, said components (71, 72) each having at least two wall segments (73) fixed to a base and extending in the direction of the central longitudinal axis (M) and arranged at a radial distance therefrom, and two wall segments (73) adjacent in a peripheral direction flanking an intermediate space, into which a wall segment (73) of the other component (71, 72) extends and is axially guided, a drive element (74) being arranged in the interior enclosed by the wall segments (73), said drive element cooperating with the second component (72) in the manner of a screw mechanism, being supported in a loading direction on the first component (71), and having a toothed rim which is arranged coaxially with the central longitudinal axis (11) and is used for rotational actuation,
- at least one wall segment (73) of the first component (72) having an access opening (75) penetrating said wall segment, the toothed rim being accessible via said access opening for rotational actuation of the drive element (74) with the aid of a manipulating tool (2).

26. Kit for implantation medicine, said kit comprising a manipulating tool (2) according to one of claims 1 to 19 and a medical implant (60) according to one of claims 20 to 25.

## Revendications

1. Outil (2)de manipulation d'un implant médical, comprenant une barre (4) d'actionnement, à l'extrémité arrière de laquelle il y a une partie (6) formant poignée et à l'extrémité avant de laquelle il y a un élément (8) d'immobilisation, qui est destiné à l'immobilisation axiale amovible de l'implant et qui a au moins deux mâchoires (10) de blocage ayant une distance radiale à l'axe (A) longitudinal médian de la barre (4) d'actionnement et - par rapport à une enveloppe (Z) cylindrique imaginaire s'étendant coaxialement à l'axe (A) longitudinal médian - une distance mutuelle dans la direction périphérique,
**caractérisé par**
un dispositif de blocage en rotation de l'outil (2) de manipulation sur un implant, dispositif qui comprend au moins un élément d'arrêt, solidaire en rotation de la barre (4) d'actionnement, coopérant d'une manière résistante à la torsion avec une surface (68) de butée de l'implant et retenu coulissant axialement sur la barre (4) d'actionnement entre une position avant, sa position de travail, et une position arrière, sa position de repos, ainsi que **caractérisé en ce que** les mâchoires de blocage servent respectivement à s'accrocher derrière au moins un élément antagoniste du côté de l'implant.

2. Outil (2) de manipulation suivant la revendication 1,
**caractérisé**
**en ce que** le au moins un élément d'arrêt est chargé par un ressort dans la direction de sa position de travail.

3. Outil (2) de manipulation suivant la revendication 1 ou 2,
**caractérisé**
**en ce que** le dispositif d'arrêt en rotation comprend un manchon (28) tubulaire qui entoure la barre (4) d'actionnement, qui coulisse axialement sur celle-ci et dont l'extrémité avant porte au moins un élément d'arrêt qui y est relié fixement.

4. Outil (2) de manipulation suivant la revendication 3,
**caractérisé par**
au moins un élément (32) de ressort, qui s'appuie par son extrémité arrière sur la barre (4) d'actionnement et par son extrémité avant sur le manchon (28) tubulaire.

5. Outil (2) de manipulation suivant la revendication 4,
**caractérisé**
**en ce que** l'extrémité arrière du manchon (28) tubulaire s'élargit radialement en formant un boîtier (34), le au moins un élément (32) de ressort étant disposé dans le boîtier (34).

6. Outil (2) de manipulation suivant l'une des revendications 3 à 5,
**caractérisé**
**en ce que** le manchon (28) tubulaire est retenu sans possibilité de se perdre à la barre (4) d'actionnement.

7. Outil (2) de manipulation suivant la revendication 6,
**caractérisé**
**en ce que** l'impossibilité de se perdre est obtenue par un élément (26) d'encliquetage chargé par un ressort sensiblement dans la direction radiale et pénétrant dans une rainure (24) périphérique avec un jeu axial, le jeu axial correspondant au trajet de déplacement entre la position de travail et la position de repos.

8. Outil (2) de manipulation suivant la revendication 7,
**caractérisé**
**en ce qu'**il y a sur le pourtour extérieur de la barre (4) d'actionnement au moins une saillie (30) annulaire sortante entre l'extrémité avant et la rainure (24) faisant le tour.

9. Outil (2) de manipulation suivant l'une des revendications 3 à 8,
**caractérisé par**
une immobilisation en rotation, composée d'un méplat et d'une saillie, le méplat (22) s'étendant axialement sur la barre (4) d'actionnement au moins sur la zone partielle avant et allant jusqu'à sa face frontale et la saillie (36) étant présente sur la face intérieure du manchon (28) tubulaire et s'appliquant au méplat (22) par un côté plat.

10. Outil (2) de manipulation suivant la revendication 9,
**caractérisé**
**en ce que** le méplat (22) est formé par le fond d'une rainure (20) axiale et la saillie (36) qui s'y étend est formée d'une manière complémentaire.

11. Outil (2) de manipulation suivant la revendication 10,
**caractérisé**
**en ce que** la au moins une saillie (36) dépasse du côté frontal du manchon (28) tubulaire et forme au moins un élément d'arrêt.

12. Outil (2) de manipulation suivant l'une des revendications précédentes,
**caractérisé**
**en ce que** les mâchoires (10) de blocage ont des bords (12) extérieurs s'éloignant de l'axe (A) longitudinal médian et ayant respectivement la même distance (R) radiale à un cercle circonscrit aux mâchoires (10) de blocage.

13. Outil (2) de manipulation suivant la revendication 12,
**caractérisé**
**en ce que** les bords (12) extérieurs des mâchoires (10) de blocage décrivent des segments d'un arc de cercle commun.

14. Outil (2) de manipulation suivant la revendication 12 ou 13,
**caractérisé**
**en ce que** sur les côtés, tournés vers l'extrémité arrière de l'outil (2) de manipulation, des mâchoires (10) de blocage, il y a des faces (14) qui coopèrent avec un élément antagoniste et qui font avec l'axe (A) longitudinal médian de la barre (4) d'actionnement un angle (α) s'ouvrant vers son extrémité avant.

15. Outil (2) de manipulation suivant la revendication 14,
**caractérisé**
**en ce que** les faces (14) sont des faces coniques se rétrécissant vers l'extrémité arrière.

16. Outil (2) de manipulation suivant l'une des revendications précédentes,
**caractérisé**
**en ce que** la barre (4) d'actionnement, y compris la partie (6) formant poignée, est traversée par un trou (16) central de réception d'un arbre (18) d'entraînement qui a, à son extrémité avant, un pignon (38) d'entraînement et, à son extrémité arrière, un bouton (40) tournant.

17. Outil (2) de manipulation suivant la revendication 16,
**caractérisé**
**en ce que** l'arbre (18) d'entraînement est monté coulissant axialement entre une position de marche à vide et une position de pénétration, le côté frontal du pignon (38) d'entraînement étant, dans la position de marche à vide, derrière axialement et, dans la position de pénétration, axialement devant le côté frontal de la barre (4) d'actionnement.

18. Outil (2) de manipulation suivant la revendication 16 ou 17,
**caractérisé**
**en ce que** la partie (16) formant poignée a, à son extrémité arrière, une rainure qui fait le tour radialement et dans laquelle pénètre, avec une complémentarité de forme efficace dans la direction axiale, un élément d'encliquetage, qui est présent dans le bouton (40) tournant et qui est soumis à l'action d'un ressort.

19. Outil (2) de manipulation suivant la revendication 17 ou 18,
**caractérisé**
**en ce que** la position de pénétration est définie par une face (50) de butée présente sur la partie (16) formant poignée et coopérant avec le bouton (40) tournant.

20. Implant (60) médical comprenant une pièce (62) ayant un axe (B) et un élément antagoniste présent sur celle-ci pour l'immobilisation amovible d'un outil de manipulation, l'élément antagoniste ayant, pour l'immobilisation en rotation de l'implant (60) par rapport à un outil (2) de manipulation, une face (68) de butée qui n'est pas orientée coaxialement à l'axe (B),
**caractérisé**
**en ce que** l'élément antagoniste a au moins deux contre-dépouilles (64) à distance de l'axe (B) et ayant respectivement une face (66) de maintien orientée dans la direction de l'intérieur de la pièce (62).

21. Implant (60) médical suivant la revendication 20,
**caractérisé**
**en ce que** la face (68) de butée comprend une face s'étendant parallèlement à l'axe (B).

22. Implant (60) médical suivant la revendication 20 ou 21,
**caractérisé**
**en ce que** les faces (66) de retenue sont formées respectivement par la paroi d'un évidement pratiqué dans la pièce (62).

23. Implant (60) médical suivant l'une des revendications 20 à 22,
**caractérisé**
**en ce que** les faces (66) de retenue font avec l'axe (B) de la pièce (62) un angle (β) aigu s'ouvrant vers l'intérieur de celle-ci.

24. Implant (60) médical suivant la revendication 23,
**caractérisé**
**en ce que** les faces (66) de retenue sont des faces partielles d'une face conique s'élargissant vers l'intérieur.

25. Implant (60) médical suivant l'une des revendications 20 à 24,
**caractérisé**
**en ce que**
- la pièce (62) est formée par une première et par une deuxième parties (71, 72) qui sont maintenues l'une contre l'autre en étant solidaires en rotation et mobiles axialement le long d'un axe (M) longitudinal médian de l'implant (60), les parties (71, 72) ayant respectivement au moins deux segments (73) de paroi, qui sont immobilisés sur une base, qui s'étendent dans la direction de l'axe (M) longitudinal médian et qui ont respectivement une distance radiale à celui-ci et respectivement deux segments (73) de paroi voisins dans la direction périphérique,
- flanque un espace intermédiaire, dans lequel un segment (73) de paroi de l'autre partie (71, 72) s'étend et y est guidé axialement, un élément (74) d'entraînement est disposé dans l'espace intérieur entouré par les segments (73) de paroi et coopère avec la deuxième partie (72) à la manière d'un mouvement à vis, s'appuie dans la direction de charge sur la première partie (71) et a une couronne dentée qui est montée coaxialement à l'axe (11) longitudinal médian et qui sert à son actionnement en rotation,
dans lequel
- au moins un segment(73) de paroi de la première partie (72) a une ouverture (75) d'accès, qui le traverse et par laquelle la roue dentée est, pour l'actionnement en rotation de l'élément (74) d'entraînement, accessible à l'aide d'un outil (2) de manipulation.

26. Jeu de pièces pour la médecine d'implantation comprenant un outil (2) de manipulation suivant l'une des revendications 1 à 19 et un implant (60) médical suivant l'une des revendications 20 à 25.
